# EUROPÄISCHE PATENTSCHRIFT

(11) **EP 1 227 854 B1**
(45) Veröffentlichungstag und Bekanntmachung des Hinweises auf die Patenterteilung: **09.03.2005**
(21) Anmeldenummer: 00987225.0
(22) Anmeldetag: 09.11.2000
(51) Int. Cl.: A61M 1/16

(54) **SOFTWARE-UPDATE FÜR EIN MEDIZINISCHES FLUIDMANAGEMENTGERÄT**
SOFTWARE UPDATE FOR A MEDICAL FLUID MANAGEMENT DEVICE
MISE A JOUR DE LOGICIEL POUR UN APPAREIL MEDICAL DE GESTION DE FLUIDE

(30) Priorität: 09.11.1999 DE 19953837
(43) Veröffentlichungstag der Anmeldung: 07.08.2002
(73) Patentinhaber: Fresenius Medical Care Deutschland GmbH, 61352 Bad Homburg v.d.H. (DE)
(72) Erfinder: BRAUER, Helge, 97469 Gochsheim (DE)
(74) Vertreter: Lorenz, Eduard
(86) Internationale Anmeldenummer: PCT/EP2000/011093
(87) Internationale Veröffentlichungsnummer: WO 2001/034222

(56) Entgegenhaltungen:
- DE-C- 19 849 787
- US-A- 5 618 441
- US-A- 5 713 856
- US-A- 5 910 139

## Beschreibung

Die vorliegende Erfindung bezieht sich auf ein medizinisches Fluidmanagementgerät mit einer Mehrzahl von Prozessorsystemen, bei dem über eine Fluidleitung Fluid zwischen einem Patienten und einer Fluidbehandlungskomponente und/oder einer Fluidquelle transportiert wird.

Unter Fluidmanagementgeräten werden hierbei insbesondere Geräte zur Leitung, Behandlung und/oder Verteilung von Flüssigkeiten und/oder Gasen verstanden.

Viele medizintechnische Geräte von höherem Komplexitätsgrad sind aus einzelnen funktionalen Modulen zusammengebaut. Dies ist zunehmend auch bei Fluidmanagementgeräten, insbesondere bei Fluidbehandlungsgeräten wie Blutbehandlungsgeräten der Fall, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispielsweise umfaßt ein modernes Hämodialysegerät eine Dialysatzubereitungs-Untereinheit, eine Ultrafiltrations-Einheit und eine Blutbehandlungs-Untereinheit.

Ein solches Blutbehandlungsgerät ist Gegenstand der DE 198 49 787 C1 der Anmelderin.

An die Betriebssicherheit medizinischer Fluidmanagementgeräte zur Behandlung von Patientenfluiden oder zum Transport eines Fluids zu einem Patienten werden naturgemäß besonders hohe Anforderungen gestellt. Dabei ist es von Vorteil, wenn das Gerät aus voneinander unabhängigen Teilkomponenten bzw. Modulen besteht, welche jeweils klar definierte Aufgaben zu erfüllen haben. Jedes Modul besitzt jeweits für sein Aufgabengebiet Ersatzsysteme und Fehlerbehandlungsabläufe, durch die sichergestellt werden kann, daß das Modul auch bei Ausfall eines anderen Systems seine Aufgabe erfüllen kann. Es hat sich gezeigt, daß eine derartige Fehlerbehandlung wesentlich zuverlässiger funktioniert als ein zentral gesteuertes System.

Ein weiterer Vorteil von modular aufgebauten Fluidmanagementgeräten ist, daß man ihre jeweilige Hardwarekonfiguration flexibel an den Einsatzzweck anpassen kann. Beispielsweise können auf Kundenwunsch zusätzliche Meß- und Überwachungsgeräte vorgesehen werden. Auch die Leistungsfähigkeit der einzelnen Module kann individuell auf die Art des Einsatzes abgestimmt werden.

Typischerweise verfügen die verschiedenen Module eines derartigen Fluidmanagementgerätes über eine Vielzahl von Mikroprozessoren. Sowohl die Meßgeräte als auch die funktionellen Einheiten sind zum großen Teil mit eigenen Prozessorsystemen ausgestattet. Jedes Prozessorsystem benötigt eine individuelle Betriebssoftware, die in einem zum Prozessorsystem gehörigen Speicher abgelegt ist.

Selbst wenn das medizinische Fluidmanagementgerät nicht aus mehreren Modulen bestehen sollte, so befinden sich aus Sicherheitsgründen oft auch in diesem Fall mindestens zwei Prozessorsysteme in dem Gerät. Ein erstes Prozessorsystem übernimmt die eigentlichen Steuerungsaufgaben, ein zweites Prozessorsystem die Oberwachungsfunktion bzgl. der Funktionsweise des ersten Prozessorsystems.

Von Zeit zu Zeit muß bei den medizinischen Fluidmanagementgeräten die Betriebssoftware ausgetauscht bzw. aktualisiert werden. Ein solche Softwareaktualisierung kann aus einer Vielzahl von Gründen erforderlich sein, wie z. B. die Beseitigung von lmplementierungsfehlem in der Software oder eine Änderung bzw. Erweiterung des Funktionsumfanges der Software z. B. durch Hinzufügen einer neuen Option. Weiterhin können sich bedingt durch Kundenwünsche oder den allgemeinen technischen bzw. medizinischen Fortschritt Änderungen an der Software eines solchen Fluidmanagementgerätes ergeben.

Hier hat nun der mehrere Prozessorsysteme aufweisende Aufbau eines medizinischen Fluidmanagementgerätes den Nachteil, daß die Betriebssoftware von jedem Prozessorsystem einzeln durch die aktuellere Version ersetzt werden muß. Daher ist es zur Durchführung einer Softwareaktualisierung bei einem modernen medizinischen Fluidmanagementgerätes erforderlich, daß speziell geschultes Fachpersonal mit einer Vielzahl von Update-Programmen an dem medizinischen Fluidmanagementgerät tätig wird, um die für die jeweilige Hardware-Konfiguration benötigten Softwareprogramme in das Gerät zu laden. Eine solche manuell durchgeführte Softwareaktualisierung ist deshalb sehr zeitaufwendig und teuer.

Diverse Veröffentlichungen aus anderen technischen Bereichen beschäftigen sich mit dem Durchführen von Softwareaktualisierungen für Prozessorsysteme. Aus der EP 0 457 940 A 1 ist ein sogenannter Aktivierungs-Schaltkreis bekannt, welcher mit einem Monitor verbunden werden kann, um einen Programm-Code oder einen Test-Code in den Monitor zu laden.

In der US 5,800,473 ist das Umprogrammieren eines Herzschrittmachers beschrieben, wobei dem Herzschrittmacher verschiedene Programme bzw. Daten in Abhängigkeit von bestimmten Parametern überspielt werden.

In der DE 44 14 597 A1 und der US 5,155,847 werden einen Zentralrechner aufweisende Computemetzwerke vorgeschlagen, wobei der Zentralrechner die Software und deren Aktualisierung der Arbeitsstationen verwaltet.

Aus der DE 44 08 544 C2 ist ein Computernetzwerk bekannt, mit deren Hilfe Zielrechner mit einem vorbereiteten Betriebssystem vorkonvektioniert werden können, das von einer Versorgungsanlage bereitgestellt wird.

Es ist die Aufgabe der vorliegenden Erfindung, eine Vorrichtung zur Verfügung zu stellen, die es erlaubt, die Softwareprogramme in einem mehrere Prozessorsysteme umfassenden medizinischen Fluidmanagementgerät auf einfache und kostengünstige Weise zu aktualisieren, ohne das dafür nur geschultes Fachpersonal in der Lage wäre. Zusätzlich soll der Sicherheitsaspekt Beachtung finden, daß eine Gefährdung eines an das medizinische Fluidmanagementgerät angeschlossenen Patienten durch den Softwareaktualisierungsvorgang wirkungsvoll und einfach vermieden werden kann.

Diese Aufgabe wird durch ein medizinisches Fluidmanagementgerät mit den Merkmalen des Patentanspruchs 1 gelöst. Vorteilhafte Ausführungsformen ergeben sich aus den Unteransprüchen.

Ein medizinisches Fluidmanagementgerät gemäß der Erfindung, z. B. eine Hämodialysemaschine, weist eine Fluidbehandlungskomponente und/oder Fluidquelle sowie eine Fluidleitung zum Transport von Fluid zwischen einem Patienten und der Fluidbehandlungskomponente bzw. Fluidquelle auf. In dem Fluidmanagementgerät sind mindestens zwei Prozessoren bzw. Prozessorsysteme enthalten, welche durch ein geeignetes System zur Datenübertragung miteinander verbunden sind. Insbesondere eignen sich dafür Bussysteme, wie z. B. ein CAN-Bus. Erfindungsgemäß ist weiter eine Dateneingabevorrichtung an das Datenübertragungssystem angeschlossen, wobei über die Dateneingabevorrichtung eine Aktualisierung der zu den jeweiligen Prozessoren gehörigen Softwareprogramme erfolgen kann. Schließlich weist das erfindungsgemäße Gerät einen Betriebsmodusschalter auf, der mit den mindestens zwei Prozessoren verbunden ist, und der in einem ersten Schaltzustand die Prozessorsysteme in einen Betriebsmodus und in einem zweiten Schaltzustand die Prozessorsysteme in einen Softwareaktualisierungsmodus schaltet. Desweiteren ist ein Erkennungssystem vorgesehen, welches ebenfalls mit dem Betriebsmodusschalter und mit dem Datenübertragungssystem verbunden ist und welches im Softwareaktualisierungsmodus unter Berücksichtigung der vorhandenen Versionen der Softwareprogramme und/oder der vorhandenen Prozessorsysteme ermittelt, welche Softwareprogramme über die Dateneingabevorrichtung für welche Prozessorsysteme zu laden sind und das Laden dieser Softwareprogramme in die betreffenden Prozessorsystemen initiiert.

Das erfindungsgemäße medizinische Fluidmanagementgerät erlaubt trotz seines mehrere Prozessorsysteme aufweisenden Aufbaus eine einfache und wenig aufwendige Aktualisierung der zu den einzelnen Prozessorsystemen gehörigen Softwareprogramme. Dabei wird durch das Erkennungssystem sichergestellt, daß lediglich die tatsächlich benötigten Softwareprogramme geladen werden. Es müssen also nicht immer alle Softwareprogramme neu geladen werden. Bei der Ermittlung, welche Softwareprogramme zu faden sind, berücksichtigt das Erkennungssystem auch die jeweilige individuelle Hardwarekonfiguration.

Der Betriebsmodusschalter bewirkt ferner eine einfache Möglichkeit, alle Prozessorsysteme gleichzeitig für eine Softwareaktualisierung zu aktivieren. Dieser Schalter ist zweckmäßig nur dann vom Betriebsmodus in den Softwareaktualisierungsmodus schaltbar, wenn die Prozessorsysteme dies aufgrund des Betriebszustandes bzgl. des Fluidmanagements zulassen. Im Softwareaktualisierungsmodus ist andererseits die Durchführung einer Fluidbehandlung bzw. eines Fluidtransportes zum Patienten unmöglich. Auf diese Weise wird eine unnötige Gefährdung des Patienten durch einen Softwareaktualisierungsvorgang vermieden.

Das medizinische Fluidmanagementgerät ist besonders vorteilshaft als Blutbehandlungsgerät wie z.B. als Hämodialysegerät einsetzbar. Bei diesem Gerät wird Blut über einen extrakorporalen Blutkreislauf dem Patienten entzogen, extrakorporat behandelt und wieder zurückgegeben. Werden diese Geräte in der Intensivmedizin verwendet, ist eine einfache Softwareaktualisierungsmöglichkeit dieser sehr komplexen Geräte zusätzlich von Vorteil, da eine schnelle und stets aktualisierte Einsatzbereitschaft garantiert werden kann.

Das erfindungsgemäße Fluidmanagementgerät kann aber auch in anderen Bereichen wie der Peritonealdialyse eingesetzt werden. Bei sogennanten Peritonealdialyse-Cyclergeräten und bei Peritonealdialysegeräten mit kontinuierlicher Dialysierflüssigkeitsumwälzung führt eine Maschine selbstständig einen zyklischen oder kontinuierlichen Austausch von Dialysierflüssigkeit im Bauchraum eines Patienten durch, wobei verwendete Dialysierflüssigkeit durch frische oder aufbereitete Dialysierfiüssigkeit ausgetauscht wird.

Peritoneal- wie Hämodialysegeräte werden auch als Heimdialysegeräte eingesetzt. Insbesondere in entlegenen Gebieten besteht hierzu kaum eine Alternative. Der Einsatz von Fachpersonal nur zur Durchführung von Softwareaktualisierung ist in diesen Fällen besonders aufwendig. Der erfindungsgemäße Aufbau des Fluidmanagementgerätes nach Anspruch 1 vereinfacht es in diesem Fall, daß auch der Patient selbst die Softwareaktualisierung vornehmen kann.

Wird als Dateneingabegerät eine Lesevorrichtung für auswechselbare Massenspeicher, wie z. B. CD-Discs, DVD-Discs oder andere Datenträger, vorgesehen, so ist es möglich, z. B. nur eine einzige CD in die Lesevorrichtung einzulegen, auf welcher alle oder eine Vielzahl von für das betreffende medizinische Fluidmanagementgerät in Frage kommenden Software-Versionen für verschiedene Geräte-Konfigurationen gespeichert sind. Jedes einzelne Prozessorsystem kann somit mittels einer einzigen CD mit einer Mehrzahl von Update-Programmen aktualisiert werden, so daß sich das separate Aktualisieren der jeweiligen Prozessorsysteme mittels separater CDs erübrigt. In diesem Fall ist es nicht mehr erforderlich, daß geschultes Fachpersonal die Aktualisierung der Software vor Ort vornehmen muß, wobei herkömmlich je nach Gerätetyp verschiedene Updates mit verschiedenen Randparametem vorgenommen werden müssen.

Die Dateneingabevorrichtung kann dabei auch ein Teil eines Prozessorsystems sein. So kann z.B. ein Prozessorsystem geeignet sein, alle Eingabe- und Ausgabeelemente wie z.B. eine Tastatur, einen Bildschirm etc. sowie die externe Datenkommunikation mittels der Dateneingabevorrichtung zu steuern.

Gemäß der Erfindung ist es möglich, vergleichsweise billig herzustellende Datenträger wie eine CD mit der jeweils aktuellsten Software-Version an die einzelnen Anwender zu verteilen, z. B. mit der Post zu verschicken, so daß ein Anwender nur die erhaltene CD bzw. einen entsprechenden Datenträger in die Lesevorrichtung einlegen muß, um die jeweils aktuellste Software-Version in sein medizinisches Fluidmanagementgerät zu laden. Dabei kann der Lade- bzw. Update-Vorgang nach erfolgtem Einlegen weitgehend automatisiert erfolgen.

Es ist auch möglich, die gesamte Aktualisierungs-Software auf einem anderen Speichermedium zu speichern, wie z. B. einem Laptop oder ähnlichem. Dieses Speichermedium muß dann mit der Dateneingabevorrichtung des medizinischen Gerätes verbunden werden, um eine gewünschte Softwareaktualisierung durchzuführen. Nach erfolgter Aktualisierung kann das Speichermedium wieder von dem medizinischen Fluidmanagementgerät getrennt bzw. entnommen werden, um ein anderes medizinisches Fluidmanagementgerät zu aktualisieren.

Weiterhin ist es möglich, daß die Aktualisierungs-Software der Dateneingabevorrichtung über Internet oder Femübertragungssignale, wie z. B. Funk oder Infrarot, übermittelt wird. Auch bei dieser Ausführungsform der Erfindung können die einzelnen Prozessorsysteme durch nur einen Vorgang jeweils auf den aktuellsten Software-Stand gebracht werden, da alle Update-Programme in einem Schritt bzw. unmittelbar nacheinander geladen werden können.

Das Erkennungssystem des erfindungsgemäßen Gerätes kann als separates Prozessorsystem oder als Teil eines bestehenden Prozessorsystems vorgesehen sein, das damit diese Aufgabe zusätzlich übernimmt. In einer besonders einem modulartigen Aufbau angepaßten Ausführungsform ist das Erkennungssystem in viele Erkennungssysteme aufgeteilt, die sich in jedem der Prozessorsysteme befinden und die nur für diese zuständig sind. Durch Aktivierung des Softwareaktualisierungsmodus erkennt dann jedes Prozessorsystem selbstständig, daß neue Software an der Dateneingabevorrichtung bereitsteht und das es seinen eigenen Aktualiserungsvorgang überprüfen und starten muß.

Gemäß einer bevorzugten Ausführungsform sind im Erkennungssystem Kennungen der vorhandenen Softwareprogramme gespeichert, wobei es sich bei diesen Kennungen insbesondere um Versionsnummern und/oder um das jeweilige Versionsdatum handeln kann. Durch Vergleich dieser Kennungen mit den Programmversionen auf dem Update-Speichermedium kann das Erkennungssystem ermitteln, ob die vorhandene Softwareversion veraltet ist oder nicht.

Gemäß einer weiteren bevorzugten Ausführungsform sind im Erkennungssystem Kennungen der vorhandenen Hardwarekonfiguration gespeichert. Durch Vergleich dieser Kennungen mit den beispielsweise per CD angebotenen Update-Softwareprogrammen können die für die jeweilige Hardwarekonfiguration geeigneten Softwareprogramme ermittelt und geladen werden. Dadurch wird ausgeschlossen, daß eine eigentlich für einen anderen Gerätetyp verwendete Software geladen wird, was hinsichtlich der hohen Sicherheitsanforderungen bei medizinischen Fluidmanagementgeräten sehr wichtig ist.

Gemäß einer weiteren bevorzugten Ausführungsform ist im Erkennungssystem eine Kennung der bevorzugten Sprachversion gespeichert. Vorteilhafterweise ist die gesamte Gerätesoftware gleich in mehreren Sprachen auf dem Speichermedium wie der CD enthalten. Durch Vergleich der Sprachkennung mit den per CD in verschiedenen Sprachversionen angebotenen Update-Softwareprogrammen kann das Erkennungssystem die für den jeweiligen Benutzer passende Sprachversion ermitteln. Über die Dateneingabevorrichtung werden dann die benötigten Softwareprogramme in der entsprechenden Sprachversion geladen.

Zum Speichern der Kennungen zur Hardware-Konfiguration, zu den vorhandenen Softwareprogrammen und zur bevorzugten Sprachversion kann z. B. ein Speicher-Chip oder eine Festplatte vorgesehen sein. Bei einem Aktualisierungsvorgang greift das Erkennungssystem auf diese Kennungen zu, wobei das Update entsprechend diesen Kennungen durchgeführt wird. Auf diese Weise wird ein vollautomatisches Update ermöglicht, welches keinerlei Fachwissen einer Bedienungsperson mehr erforderlich macht. Dies entspricht sozusagen einer sogenannten "plug-and-play"-Funktion des Softwareaktualisierungsvorgangs selbst: Nachdem z.B. eine CD mit der aktuellen Software in die Dateneingabevorrichtung eingelegt und der Betriebsmodusschalter in den Softwareaktualiserungsmodus geschaltet wurde, laufen die Softwareaktualisierungsprozesse automatisiert ab, ohne daß es eines weiteren Eingreifens bedarf.

Vorteilhaft kann bei einer Aktualisierung der Software auch die Dokumentation der Software bzw. des medizinischen Fluidmanagementgerätes durch auf dem Datenträger gespeicherte Informationen geändert bzw. angepaßt werden. Die Aktualiserung der Dokumentationsdaten erfolgt dabei ebenfalls über die Dateneingabevorrichtung. Auf diese Weise können nach erfolgtem Update die für einen Benutzer nützlichen Informationen bezüglich der neuen Software zur Verfügung gestellt werden. Dies kann z. B. durch Ausgabe entsprechender Informationen auf einer Anzeigevorrichtung wie einem meist ohnehin vorhandenen Bildschirm erfolgen, wobei ein Benutzer durch entsprechend kommentierte Menüführung leicht mit der aktuatisierten Software arbeiten kann. Insbesondere können Informationen über neue Leistungmerkmale ausgegeben werden. Weiterhin können auf dem Datenträger auch Informationen bezüglich technischer Unterlagen, Manuals, verfügbarer Ersatzteile oder ähnliches gespeichert sein. Durch das gleichzeitige Laden der aktuellen Softwareprogramme und der zugehörigen Dokumentation wird darüberhinaus sichergestellt, daß der Benutzer jeweils die aktuelle Dokumentation zu Rate zieht. Auch dies ist wegen der Sicherheitsaspekte insbesondere bei medizinischen Fluidmanagementgeräten von Bedeutung.

Bei einer weiteren vorteilhaften Ausgestaltung der Erfindung werden die Dokumentationsdaten in der vom Benutzer bevorzugten Sprachversion geladen. Die bevorzugte Sprache kann anhand der im Erkennungssystem gespeicherten Kennung ermittelt werden.

Vorteilhaft ist eine Anzeigevorrichtung bei dem medizinischen Fluidmanagementgerät vorgesehen, welche eine Hardware-Konfiguration und/oder Versions-Information der verwendeten Software darstellen kann. Diese Anzeigevorrichtung ist entweder Teil eines bestehenden Prozessorsystems oder direkt an das Datenübertragungssystem angeschlossen. Zum Beispiel kann mittels eines Bildschirms dargestellt werden, welche Prozessorsysteme tatsächlich in welcher Konfiguration in dem medizinischen Fluidmanagementgerät enthalten sind und welches die verwendeten Software-Versionen sind, wobei z. B. aus einem Speicher die Datumsangaben bzw. Aktualisierungsdaten der jeweils letzten erfolgten Softwareaktualiserungen abgerufen und angezeigt werden können.

Es ist von Vorteil, wenn die Anzeigevorrichtung geeignet ist, den Ladevorgang betreffende Informationen ausgeben zu können. Außerdem kann das medizinische Fluidmanagement Bestätigungsmittel zum Bestätigen des Ladevorgangs bzw. von Teilen des Ladevorgangs umfassen, die Teil eines Prozessorsystems oder direkt an das Datenübertragungssystem angeschlossen sind.

Die Erfindung wird nachfolgend beispielhaft anhand zweier Ausführungsformen beschrieben. Es zeigen:
- Figur 1: ein vereinfachtes Blockschaltbild einer ersten Ausführungsform eines erfindungsgemäßen medizinischen Fluidmanagementgerätes mit einer Mehrzahl von Prozessorsystemen;
- Figur 2: ein vereinfachtes Blockschaltbild einer zweiten Ausführungsform eines erfindungsgemäßen medizinischen Fluidmanagementgerätes mit einer Mehrzahl von Prozessorsystemen.

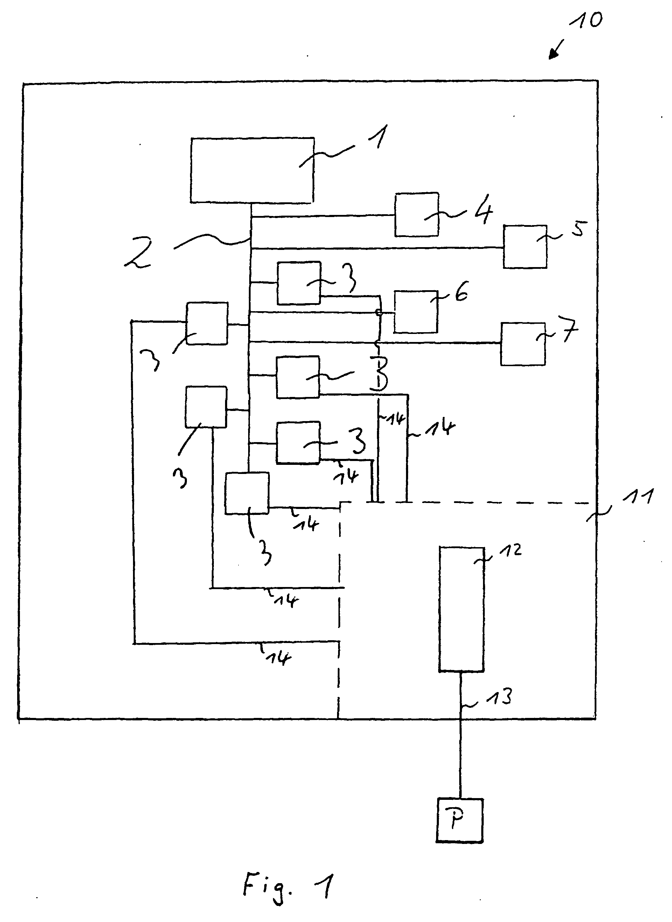

In Figur 1 ist eine erste Ausführungsform eines medizinischen Fluidmanagementgerätes 10 nach der Erfindung dargestellt. Dabei sind nur die Komponenten schematisch gezeigt, die für das Verständnis der Erfindung relevant sind. Die weitere Gestattung derartiger Geräte sind dem mit derartigen Geräten vertrauten Fachmann geläufig. Beispielsweise zeigt die deutsche Patentanmeldung 198 49 787 der Anmelderin ein solches Gerät als Hämodialysegerät, auf deren Offenbarung hiermit ausdrücklich Bezug genommen wird.

Das medizinische Fluidmanagementgerät, beispielsweise ein Fluidbehandlungsgerät wie ein Hämodialysegerät, weist mehrere Prozessorsysteme 3 auf, welche miteinander durch ein Datenübertragungssystem 2 verbunden sind. Mit dem Datenübertragungssystem 2 ist eine Dateneingabevorrichtung 1 sowie eine Software/Hardware-Erkennungsvorrichtung 4 verbunden.

Des weiteren ist ein Betriebsmodusschalter 5 mit dem Datenübertragungssystem 2 und damit mit den Prozessorsystemen 3 verbunden. Der Betriebsmodusschalter hat mindestens einen ersten und einen zweiten Schaltzustand, die einen Betriebsmodus bzw. einen Softwareaktualisierungsmodus definieren. Der Betriebsmodusschalter 5 ist dabei als manueller Schalter ausgebildet. Es ist aber auch möglich, den Betriebsmodusschalter 5 als Teil der Software zu realisieren, d.h. einen Wechsel zwischen den beiden Schaltzuständen als Teil eines ablaufenden Programmes zu gestalten. Es kann auch vorgesehen sein, daß der Betriebsmodusschalter als Femsteuerung konzipiert ist. In diesem Fall sollten vor Ort zusätzliche Bestätigungsmittel vorhanden sein.

An das Datenübertragungssystem 2 sind außerdem eine Anzeigevorrichtung 6 sowie Bestätigungsmittel 7 angeschlossen.

Symbolisch ist außerdem ein Teil 11 des Fluidmanagementgerätes 10 dargestellt, der eine Fluidbehandlungskomponente 12 zeigt, die über eine Fluidleitung 13 mit einem Patienten P verbunden ist. Im Falle eines Hämodialysegerätes ist die Fluidbehandlungskomponente 12 ein Hämodialysator und/oder ein Hämofilter. Die Fluidleitung 13 stellt dann das extrakorporale Schlauchsystem dar, mit dem Blut vom Patienten P zur Blutbehandlungskomponente 12 und zurück transportiert wird.

Die Prozessorsysteme 3 sind mit dem Teil 11 des Fluidmanagementgerätes 10 über Leitungen 14 verbunden, die zu nicht näher gezeigten Sensoren und/oder Aktoren führen. Diese Sensoren und/oder Aktoren überwachen und/oder steuern die Fluidbehandlung bzw. den Fluidtransport. Wiederum im Falle eines Hämodialysegerätes überwachen und/oder steuern diese Sensoren und/oder Aktoren den Transport des Blutes von dem Patienten zur Blutbehandlungskomponente 12 und zurück sowie die eigentliche Blutbehandlung selbst. Bzgl. von Beispielen in diesem Fall für diese Sensoren und/oder Aktoren wird ausdrücklich auf das in der DE 198 49 787 C1 offenbarte medizinische Blutbehandlungsgerät mit mehreren Prozessorsystemen Bezug genommen.

Es ist auch denkbar, daß die Leitungen 14 als Teil des Datenübertragungssystems 2 ausgestaltet sind, d.h. die Sensoren und/oder Aktoren werden von den Prozessorsystemen 3 über das Datenübertragungssystem 2 angesteuert bzw. ausgelesen.

Die Dateneingabevorrichtung 1 ist in dieser Ausführungsform als CD-Laufwerk ausgebildet. Wird von einem Benutzer eine Softwareaktualisierungs-CD in die Dateneingabevorrichtung 1 eingelegt und der Betriebsmodusschalter 5 in den Softwareaktualisierungsmodus geschaltet, so wird der Softwareaktualisierungsvorgang von dem Erkennungssystem 4 automatisch gestartet. Gleichzeitig gehen die Prozessorsysteme 3 in einen Wartezustand, um ggf. mit einer neuen Betriebssoftware versehen zu werden. Dabei können z. B. von dem Erkennungssystem 4 zunächst Daten bezüglich der auf dem Datenträger gespeicherten Software-Versionen von der CD ausgelesen werden, welche von der Erkennungsvorrichtung 4 mit der bei ihr gespeicherten tatsächlichen Konfiguration des medizinischen Fluidmanagementgerätes verglichen werden, um festzustellen, ob sich Updates auf tatsächlich in dem Gerät eingesetzten Prozessorsystemen bzw. deren Konfiguration beziehen. Weiterhin kann durch einen Vergleich der aus dem Datenträger ausgelesenen Daten mit in der Erkennungsvorrichtung 4 eingespeicherten Daten ermittelt werden, ob die von den Prozessorsystemen 3 verwendeten Software-Versionen noch aktuell sind oder aktualisiert werden müssen.

Wird dies festgestellt, so kann in einem zweiten Schritt der eigentliche Update-Vorgang von dem Erkennungssystem 4 initiiert werden, wobei die jeweils aktuellsten Software-Versionen der noch eine ältere Software verwendenden Prozessorsysteme 3 heruntergeladen und in diese Prozessorsysteme 3 eingespielt werden. Somit kann z. B. unter Verwendung nur einer einzigen CD das gesamte aus verschiedenen Prozessorsystemen 3 bestehende System mit einer Vielzahl von unterschiedlichen Software-Versionen mit einem einzigen Aktualisierungs-Vorgang auf den jeweils neuesten Stand gebracht werden, wofür kein speziell ausgebildetes Fachpersonal mehr notwendig ist.

Auf der Anzeigevorrichtung 6, die einen bei vielen Geräten sowieso vorhandenen Bildschirm darstellt, werden dem Anwender Informationen über den Ladevorgang gegeben. insbesondere ist vorgesehen, daß jeder Überprüfungsvorgang der einzelnen Prozessorsysteme 3 dargestellt wird und daß die Einspielung der jeweils neuen Software erst dann vorgenommen wird, wenn der Anwender dies mit Hilfe der Bestätigungsmittel 7 quittiert hat. Als Bestätigungsmittel kann ein zusätzliches Eingabemittel vorgesehen sein, es ist aber auch möglich, auf eine oft vorhandene Tastatur zurückzugreifen.

Wird das medizinische Fluidmanagementgerät 10 am Ende des Softwareaktualisierungsvorgangs wieder mit dem Betriebsmodusschalter 6 in den Betriebsmodus geschaltet, so steht das aktualisierte System umgehend z.B. für eine Blutbehandlung bereit. Ggf. kann dabei zunächst ein automatisch gestarteter Initialisierungsprozeß notwendig sein.

Der Betriebsmodusschalter 6 kann aus Sicherheitsgründen nur dann in den Softwareaktualisierungsmodus geschaltet werden, wenn die Prozessorsysteme 3 dies aufgrund ihres Status erlauben. Hierdurch soll verhindert werden, daß ein Aktualisierungsvorgang vorgenommen wird, während ein Patient an das Fluidmanagementgerät angeschlossen ist und der Transport von Fluid in und/oder aus dem Patienten in Gange ist.

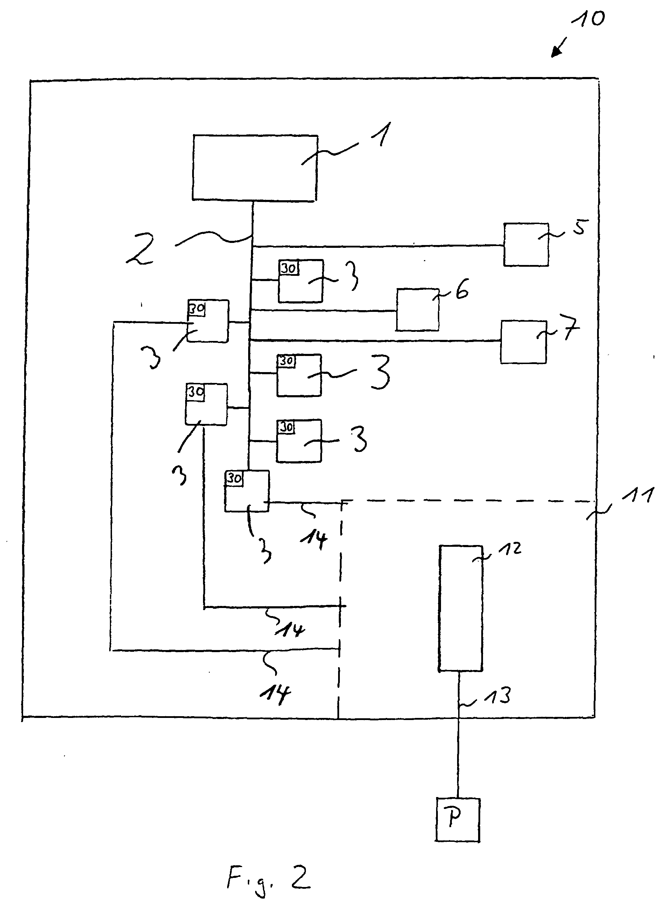

Die Figur 2 zeigt eine andere Ausführungsform eines erfindungsgemäßen medizinischen Fluidmanagementgerätes 10, das in großen Teilen der in der Figur 1 gezeigten Ausführungsform entspricht. Aus diesem Grunde wurden in beiden Figuren gleiche Bestandteile mit der gleichen Bezugsziffer versehen. Der Unterschied beider Ausführungsformen liegt in einer anderen Umsetzung des Erkennungssystems 4, das in Figur 2 als Teil 30 eines jeden Prozessorsystems 3 dargestellt ist. Bei dieser Ausführungsform ist damit auch das Erkennungssystem dezentralisiert.

Schaltet ein Anwender in diesem Fall den Softwareaktualiserungsmodus ein, so wird dies den Erkennungssystemen 30 wiederum über das Datenübertragungssystem 2 mitgeteilt. Diese organisieren nun vollkommen selbstständig den Ablauf der Aktualisierung der Betriebssoftware ähnlich wie in der oben angegebenen Art und Weise. In diesem Fall ist jedes Erkennungssystem 30 aber nur für sein Prozessorsystem 3 zuständig. Bei dieser Ausführungsform ist das Datenübertragungssystem 2 geeignet, einen eigenständigen Zugriff der Prozessorsysteme 3 auf die Dateneingabevorrichtung 1 zuzulassen, wie es insbesondere bei einem CAN-Bus möglich ist. Diese Ausführungsform hat den Vorteil, daß die Aktualisierungsfunktion nicht vom Funktionieren eines einzelnen Erkennungssystems abhängt.

Im übrigen sei darauf hingewiesen, daß auch allerlei Abwandlungen der Gestaltung des Erkennungssystems 4 bzw. 30 im Sinne von Mischformen möglich sind, ohne daß der Idee der beanspruchten Erfindung verlassen wird. So kann das Erkennungssystem 4 direkt Teil eines bestimmten Prozessorsystems 3 sein. Die Verwaltung der Versionsnummern und/oder Daten, bzw. Konfigurationsinformation muß in diesem Fall nicht zentral bei dem einen Erkennungssystem 4 hinterlegt sein. Diese kann lokal bei jedem Prozessorsystem 3 gespeichet sein, wobei sie jeweils einzeln von dem einen Erkennungssystem 4 abgefragt wird. Genausogut ist es möglich, daß die Erkennungssysteme 30 dezentralisiert sind, aber alle auf einen gemeinsamen Datenspeicher zugreifen, der in dem medizinischen Fluidmanagementgerät 10 enthalten und mit dem Datenübertragungssystem 2 verbunden ist. Dieser kann für den Bootvorgang des Fluidmanagementgerätes 10 nach dem Einschalten sowieso notwendig sein, falls keine permanente Programmspeicher wie EPROMs zur Verfügung stehen und die gesamte Software jedes Prozessorsystems in dieser Phase zunächst geladen werden muß.

## Patentansprüche

1. Medizinisches Fluidmanagementgerät mit
a) einer Fluidbehandlungskomponente und/oder Fluidquelle;
b) einer Fluidleitung zum Transport von Fluid zwischen einem Patienten und der Fluidbehandlungskomponente und/oder Fluidquelle;
c) mindestens zwei Prozessorsystemen (3) mit jeweils zugehörigen Softwareprogrammen;
d) einem Datenübertragungssystem (2) zum Verbinden der Prozessorsysteme (3);
e) einer Dateneingabevorrichtung (1), die mit dem Datenübertragungssystem (2) verbunden ist und über die eine Aktualisierung der zu den jeweiligen Prozessorsystemen (3) gehörigen Softwareprogramme erfolgen kann;
f) einem Betriebsmodusschalter (5), der mit den mindestens zwei Prozessorsystemen (3) verbunden ist, und der in einem ersten Zustand die Prozessorsysteme (3) in einen Betriebsmodus und in einem zweiten Zustand die Prozessorsysteme (3) in einen Softwareaktualisierungsmodus schaltet; sowie
g) einem Erkennungssystem (4, 30), welches ebenfalls mit dem Betriebsmodusschalter (5) und mit dem Datenübertragungssystem (2) verbunden ist und im Softwareaktualisierungsmodus unter Berücksichtigung der vorhandenen Versionen der Softwareprogramme und/oder der vorhandenen Prozessorsysteme (3) ermittelt, welche Softwareprogramme über die Dateneingabevorrichtung (1) für welche Prozessorsysteme (3) zu laden sind und das Laden dieser Softwareprogramme in die betreffenden Prozessorsysteme initiiert.

2. Medizinisches Fluidmanagementgerät nach Anspruch 1, **dadurch gekennzeichnet, daß** das Fluid Blut ist.

3. Medizinisches Fluidmanagementgerät nach Anspruch 2, **dadurch gekennzeichnet, daß** die Fluidbehandlungskomponente ein Hämodialysator und/oder ein Hämofilter ist.

4. Medizinisches Fluidmanagementgerät nach einem der vorhergehenden Ansprüche, **dadurch gekennzeichnet, daß** das Erkennungssystem (4, 30) Teil eines Prozessorsystems (3) ist.

5. Medizinisches Fluidmanagementgerät nach einem der Ansprüche 1 bis 3, **dadurch gekennzeichnet, daß** jedes der mindestens zwei Prozessorsysteme (3) sein eigenes Erkennungssystem (30) aufweist.

6. Medizinisches Fluidmanagementgerät nach einem der vorhergehenden Ansprüche, **dadurch gekennzeichnet, daß** die Dateneingabevorrichtung (1) eine Lesevorrichtung für einen auswechselbaren Datenspeicher, insbesondere für CDs oder DVDs ist.

7. Medizinisches Fluidmanagementgerät nach einem der Ansprüche 1 bis 5, **dadurch gekennzeichnet, daß** die Dateneingabevorrichtung (1) eine Empfangsvorrichtung oder Schnittstelle zum Empfangen von extern gespeicherten Daten ist.

8. Medizinisches Fluidmanagementgerät nach einem der vorhergehenden Ansprüche, **dadurch gekennzeichnet, daß** das Erkennungssystem (4, 30) eine Vorrichtung zum Speichern von Kennungen der vorhandenen Softwareprogramme umfaßt, wobei es sich bei den Kennungen insbesondere um Versionsnummern und/oder um das jeweilige Versionsdatum handeln kann.

9. Medizinisches Fluidmanagementgerät nach einem der vorhergehenden Ansprüche, **dadurch gekennzeichnet, daß** das Erkennungssystem (4, 30) eine Vorrichtung zum Speichern einer Kennung der vorhandenen Hardwarekonfiguration umfaßt.

10. Medizinisches Fluidmanagementgerät nach einem der vorhergehenden Ansprüche, **dadurch gekennzeichnet, daß** das Erkennungssystem (4, 30) eine Vorrichtung zum Speichern einer Kennung der bevorzugten Sprachversion umfaßt.

11. Medizinisches Fluidmanagementgerät nach einem der vorhergehenden Ansprüche, **dadurch gekennzeichnet, daß** das Erkennungssystem (4, 30) ermittelt, welche Sprachversion der benötigten Softwareprogramme über die Dateneingabevorrichtung (1) zu laden ist.

12. Medizinisches Fluidmanagementgerät nach einem der vorhergehenden Ansprüche, **dadurch gekennzeichnet, daß** über die Dateneingabevorrichtung (1) eine Aktualisierung der zu den benötigten Softwareprogrammen gehörigen Dokumentationsdaten erfolgen kann.

13. Medizinisches Fluidmanagementgerät nach Anspruch 12, dadurch gekenneichnet, daß das Erkennungssystem (4,30) ermittelt, welche Sprachversion der benötigten Dokumentationsdaten über die Dateneingabevorrichtung (1) zu laden ist.

14. Medizinisches Fluidmanagementgerät nach einem der vorhergehenden Ansprüche, **dadurch gekennzeichnet, daß** das medizinische Fluidmanagementgerät ferner eine Anzeigevorrichtung (6) umfaßt, die Teil eines Prozessorsystems (3) oder direkt an das Datenübertragungssystem (2) angeschlossen ist.

15. Medizinisches Fluidmanagementgerät nach Anspruch 14, **dadurch gekennzeichnet, daß** die Anzeigevorrichtung (6) geeignet ist, Informationen über den Ladevorgang auszugeben.

16. Medizinisches Fluidmanagementgerät nach Anspruch 14 oder 15, **dadurch gekennzeichnet, daß** die Anzeigevorrichtung (6) geeignet ist, bzgl. der Ladevorgänge der einzelnen Prozessorsysteme (3) eine Bestätigungsanfrage anzuzeigen und das medizinische Fluidmanagementgerät ferner Bestätigungsmittel (7) umfaßt, die Teil eines Prozessorsystems (3) oder direkt an das Datenübertragungssystem (2) angeschlossen sind, mit denen die einzelnen Ladevorgänge von einem Anwender vor dem Laden bestätigt werden müssen.

## Claims

1. Medical fluid management appliance having
a) a fluid handling component and/or fluid source;
b) a fluid line for transporting fluid between a patient and the fluid handling component and/or fluid source;
c) at least two processor systems (3) having respective associated software programs;
d) a data transmission system (2) for connecting the processor systems (3);
e) a data input apparatus (1) which is connected to the data transmission system (2) and can be used to update the software programs associated with the respective processor systems (3);
f) an operating mode switch (5) which is connected to the at least two processor systems (3) and, in a first state, switches the processor systems (3) to one mode of operation and, in a second state, switches the processor systems (3) to a software update mode; and
g) an identification system (4, 30) which is likewise connected to the operating mode switch (5) and to the data transmission system (2) and, in the software update mode, takes into account the existing versions of the software programs and/or the existing processor systems (3) in order to ascertain which software programs need to be loaded using the data input apparatus (1) for which processor systems (3), and initiates loading of these software programs into the processor systems in question.

2. Medical fluid management appliance according to Claim 1, **characterized in that** the fluid is blood.

3. Medical fluid management appliance according to Claim 2, **characterized in that** the fluid handling component is a haemodialyser and/or a haemofilter.

4. Medical fluid management appliance according to one of the preceding claims, **characterized in that** the identification system (4, 30) is part of a processor system (3).

5. Medical fluid management appliance according to one of Claims 1 to 3, **characterized in that** each of the at least two processor systems (3) has its own identification system (30).

6. Medical fluid management appliance according to one of the preceding claims, **characterized in that** the data input apparatus (1) is a reading apparatus for a replaceable data store, particularly for CDs or DVDs.

7. Medical fluid management appliance according to one of Claims 1 to 5, **characterized in that** the data input apparatus (1) is a reception apparatus or an interface for receiving externally stored data.

8. Medical fluid management appliance according to one of the preceding claims, **characterized in that** the identification system (4, 30) comprises an apparatus for storing identifiers for the existing software programs, the identifiers possibly being version numbers and/or the respective version date, in particular.

9. Medical fluid management appliance according to one of the preceding claims, **characterized in that** the identification system (4, 30) comprises an apparatus for storing an identifier for the existing hardware configuration.

10. Medical fluid management appliance according to one of the preceding claims, **characterized in that** the identification system (4, 30) comprises an apparatus for storing an identifier for the preferred language version.

11. Medical fluid management appliance according to one of the preceding claims, **characterized in that** the identification system (4, 30) ascertains which language version of the requisite software programs needs to be loaded using the data input apparatus (1).

12. Medical fluid management appliance according to one of the preceding claims, **characterized in that** the data input apparatus (1) may be used to update the documentation data associated with the requisite software programs.

13. Medical fluid management appliance according to Claim 12, **characterized in that** the identification system (4, 30) ascertains which language version of the requisite documentation data needs to be loaded using the data input apparatus (1).

14. Medical fluid management appliance according to one of the preceding claims, **characterized in that** the medical fluid management appliance also comprises a display apparatus (6) which is part of a processor system (3) or is connected directly to the data transmission system (2).

15. Medical fluid management appliance according to Claim 14, **characterized in that** the display apparatus (6) is suitable for outputting information about the loading operation.

16. Medical fluid management appliance according to Claim 14 or 15, **characterized in that** the display apparatus (6) is suitable for displaying a confirmation request for the loading operations of the individual processor systems (3), and the medical fluid management appliance also comprises confirmation means (7) which are part of a processor system (3) or are connected directly to the data transmission system (2) and need to be used by a user to confirm the individual loading operations before the loading.

## Revendications

1. Appareil médical de gestion de fluide avec
a) un composant de traitement de fluide et/ou une source de fluide;
b) un conduit de fluide pour le transport du fluide entre un patient et le composant de traitement de fluide et/ou la source de fluide;
c) au moins deux systèmes de processeur (3) avec des programmes de logiciel respectivement associés;
d) un système de transmission de données (2) pour relier les systèmes de processeur (3);
e) un dispositif d'entrée des données (1) qui est relié au système de transmission de données (2) et par lequel peut avoir lieu une actualisation des programmes de logiciel associés aux systèmes de processeur respectifs (3);
f) un commutateur de mode de fonctionnement (5) qui est relié à au moins deux systèmes de processeur précités (3) et qui commute dans un premier état les systèmes de processeur (3) en un mode de fonctionnement, et qui commute dans un second état les systèmes de processeur (3) en un mode d'actualisation du logiciel; et
g) un système de reconnaissance (4, 30) qui est également relié au commutateur de mode de fonctionnement (5) et au système de transmission des données (2) et qui détermine en mode d'actualisation du logiciel, en tenant compte des versions existantes des programmes de logiciel et/ou des systèmes de processeur existants (3), les programmes de logiciel à charger par le dispositif d'entrée de données (1) pour les systèmes de processeur concernés (3) et qui initie le chargement de ces programmes de logiciel dans les systèmes de processeur concernés.

2. Appareil médical de gestion de fluide selon la revendication 1, **caractérisé en ce que** le fluide est du sang.

3. Appareil médical de gestion de fluide selon la revendication 2, **caractérisé en ce que** le composant de traitement de fluide est un hémodialyseur et/ou un hémofiltre.

4. Appareil médical de gestion de fluide selon l'une des revendications précédentes, **caractérisé en ce que** le système de reconnaissance (4, 30) fait partie d'un système de processeur (3).

5. Appareil médical de gestion de fluide selon l'une des revendications 1 à 3, **caractérisé en ce que** chacun d'au moins deux systèmes de processeur précités (3) possède son propre système de reconnaissance (30).

6. Appareil médical de gestion de fluide selon l'une des revendications précédentes, **caractérisé en ce que** le dispositif d'entrée de données (1) est un dispositif de lecture d'une mémoire de données échangeable, en particulier pour des CDs ou des DVDs.

7. Appareil médical de gestion de fluide selon l'une des revendications 1 à 5, **caractérisé en ce que** le dispositif d'entrée des données (1) est un dispositif de réception ou une interface pour recevoir des données stockées à l'extérieur.

8. Appareil médical de gestion de fluide selon l'une des revendications précédentes, **caractérisé en ce que** le système de reconnaissance (4, 30) comprend un dispositif de stockage d'indicatifs des programmes de logiciel existants, où dans le cas des indicatifs, il peut s'agir en particulier de numéros de version et/ou de la date de version respective.

9. Appareil médical de gestion de fluide selon l'une des revendications précédentes, **caractérisé en ce que** le système de reconnaissance (4, 30) comprend un dispositif de stockage d'un indicatif de la configuration de matériel existante.

10. Appareil médical de gestion de fluide selon l'une des revendications précédentes, **caractérisé en ce que** le système de reconnaissance (4, 30) comprend un dispositif de stockage d'un indicatif de la version de langue préférée.

11. Appareil médical de gestion de fluide selon l'une des revendications précédentes, **caractérisé en ce que** le système de reconnaissance (4, 30) détermine la version de langue des programmes de logiciel requis qui est à charger par le dispositif d'entrée de données (1).

12. Appareil médical de gestion de fluide selon l'une des revendications précédentes, **caractérisé en ce que** par le dispositif d'entrée de données (1), une actualisation des données de documentation associées aux programmes de logiciel requis peut avoir lieu.

13. Appareil médical de gestion de fluide selon la revendication 12, **caractérisé en ce que** le système de reconnaissance (4,30) détermine la version de langue des données de documentation requises qui est à charger par le dispositif d'entrée de données (1).

14. Appareil médical de gestion de fluide selon l'une des revendications précédentes, **caractérisé en ce que** l'appareil médical de gestion de fluide comprend en outre un dispositif d'affichage (6) qui fait partie d'un système de processeur (3) ou qui est relié directement au système de transmission de données (2).

15. Appareil médical de gestion de fluide selon la revendication 14, **caractérisé en ce que** le dispositif d'affichage (6) convient pour émettre des informations se rapportant à l'opération de charge.

16. Appareil médical de gestion de fluide selon la revendication 14 ou 15, **caractérisé en ce que** le dispositif d'affichage (6) convient, en ce qui concerne les opérations de charge des systèmes de processeur individuels (3), à afficher une demande de confirmation, et que l'appareil médical de gestion de fluide comprend en outre des moyens de confirmation (7) qui font partie d'un système de processeur (3) ou qui sont directement reliés au système de transmission de données (2), au moyen desquels les opérations de charge individuelles doivent être confirmées par un utilisateur avant la charge.
